(19) Europäisches Patentamt
European Patent Office
Office européen des brevets

(11) **EP 3 051 280 A1**

(12) **EUROPEAN PATENT APPLICATION**

(43) Date of publication:
**03.08.2016 Bulletin 2016/31**

(51) Int Cl.:
**G01N 27/12** (2006.01)

(21) Application number: **15000250.9**

(22) Date of filing: **28.01.2015**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br><br>(71) Applicant: **Sensirion AG**<br>**8712 Stäfa (CH)** | (72) Inventor: **Böni, Dominic**<br>**CH-8712 Stäfa (CH)**<br><br>(74) Representative: **Toleti, Martin**<br>**c/o E.Blum & Co. AG**<br>**Vorderberg 11**<br>**8044 Zürich (CH)** |

(54) **Metal oxide based gas sensor with pulsed heating**

(57) A concentration of a compound in a gas is determined by a gas sensor (10) with a layer (11) comprising metal oxide. The layer (11) is heated by first heating pulses (H1) of a first duration (T1) each, and by at least one second heating pulse (H2) of a second duration (T2) between two consecutive first heating pulses (H1), which second duration (T2) is less than the first duration (T1) and is equal to or less than 5 seconds. The concentration of the gas compound is derived from values indicative of a resistance of the layer (11) as measured in response to one or more of the first and second heating pulses (H1, H2).

FIG. 3

**Description**

FIELD OF THE INVENTION

The present invention relates to a method for determining a concentration of a compound in a gas, and to a gas sensor, particularly a gas sensor using metal oxide.

BACKGROUND OF THE INVENTION

Metal oxide gas sensors are based on the concept that gaseous components - also referred to as analytes - interact with a layer comprising the metal oxide at elevated temperatures of the layer in the range of more than 100° Celsius. As a result of a catalytic reaction, a conductivity or resistance of the layer may change which change can be measured. Hence, such gas sensors are also denoted as high temperature chemoresistors for the reason that a property of the compound is converted into an electrical resistance at high temperatures of the layer.

A gas sensor can be a gas sensor for detecting one or more compounds in a gas, which gas specifically may be air surrounding a portable multi-purpose electronic device containing the gas sensor such as a mobile phone or a tablet computer. Hence, in a sample application it may be of interest to identify, if such air may contain compounds the gas sensor is tuned to detect.

Specifically when being arranged in a portable electronic device, it is desired that the gas sensor operates within confines set by power available in such a device.

SUMMARY OF THE INVENTION

According to a first aspect of the invention, a method is provided for determining a concentration of a compound in a gas by using a gas sensor. The gas sensor has a layer comprising metal oxide. The layer is heated, preferably by means of a heater of the gas sensor. A heating pattern is applied comprising first heating pulses of a first duration each, and at least one second heating pulse of a second duration, between two consecutive first heating pulses, which second duration is less than the first duration and is equal to or less than 5 seconds. Preferably, more than one second heating pulse is applied between two consecutive first heating pulses, such as five heating pulses, for example. The concentration of the gas compound is derived from values indicative of a resistance of the layer as measured in response to one or more of the first and second heating pulses.

According to a second aspect of the present invention, a gas sensor is provided comprising a layer comprising metal oxide which layer preferably is arranged between two electrodes. A heater is provided preferably comprising one or more heating elements arranged to heat the layer. In a preferred embodiment, the gas sensor comprises a chip with a substrate such as a semiconductor substrate, which is thinned in a dedicated region for generating a membrane that is thermally insulated from the rest of the chip and that is also referred to as hotplate. Preferably, the heater and the layer that is sensitive to one or more gas components are arranged on such membrane. Further, a control unit is provided for controlling the heater to heat the layer by the first heating pulses and the second heating pulses as explained in connection with the method. The control unit further is adapted to derive the concentration.

Hence, a pulsed heating pattern is applied comprising first heating pulses and in between one or more second heating pulses of a duration shorter than the first heating pulses. This drastically reduces power consumption compared to a constant heating over a longer period in time. Accurate measurements are guaranteed in view of the regular first heating pulses which are typically dimensioned with respect to their duration such that an exact measurement can be taken during or in response to the heating pulse. However, in order to remain in power consumption limits possibly given for certain applications such as smartphones, wearables, battery driven devices, etc. the refresh rate of such first heating pulses is reduced without waiving measurement results since in between two consecutive first heating pulses one or more second heating pulses are generated of a shorter duration each than one of the first heating pulses. Preferably, the duration of a second heating pulse is half or less than the duration of a first heating pulse, and preferably is twenty percent or less. Hence, a hybrid mode is introduced including generating first and second heating pulses for heating the layer in order to enable measurements of e.g. the resistance of the layer or a value indicative of the resistance of the layer, such as a voltage drop at across the layer, at different terms. This allows achieving a fast refresh rate of concentration values while at the same time spending only little power and receiving high accuracy results.

Given that heating pulses of different duration are to be generated by means of the heater for acting on the layer, it is preferred that corresponding electrical pulses are applied to the heater for generating a heating pulse each. It is assumed that an electrical pulse such as an electrical current pulse applied to the heater results in a heating pulse of more or less the same duration. Hence, for generating one of the first heating pulses, the heater is activated by a first electrical pulse of the first duration. Accordingly, for generating one of the second heating pulses, the heater is activated by a second electrical pulse of the second duration. It is preferred, that the first and the second electrical pulse each show the same magnitude, or at least differ in magnitude by a maximum of thirty percent from the lower of the first and the second magnitude.

Preferably, the heater is inactive between a first heating pulse and a subsequent second heating pulse, and / or between two consecutive second heating pulses. Preferably, the heater is inactive outside the second heating pulses within two consecutive first heating pulses. Alternatively, the heater may be activated during these times, however at a low power. For example, the heater may be activated at a maximum of twenty percent, and preferably ten percent, of the lower of the first and the second magnitude. By such means, power may be saved given that in between the first and / or second heating pulses no or only little power is consumed by the heater.

The heating may be performed open-loop, i.e. without a feedback from the layer to be heated. In such embodiment, the sequence of the first and second electrical pulses may be stored in a memory of the control unit and result in the desired heating pattern upon execution. In a different embodiment, the heating is performed closed-loop including a feedback from the layer to the control unit. For example, a temperature sensor in or close to the layer, e.g. arranged in the membrane, may provide present temperature values to the control unit. In the control unit, a temperature pattern may be stored representing a temperature profile achieved in response to the sequence of first and second heating pulses. Hence, the temperature in the layer may be controlled to the desired profile. For this purpose, a deviation of a currently measured temperature value from the desired temperature profile value is translated into a control signal for the heater.

The duration of the or each first heating pulse, which is also referred to as first duration, preferably is equal to or less than 10 minutes, and in particular is equal to or less than 1 minute, and in particular is equal to or less than 10 seconds, and in particular is equal to or less than 1 second, and in particular is equal to or less than 500 milliseconds, and in particular is equal to or less than 150 milliseconds. Preferably, the first duration is in a range between 100 milliseconds and 30 seconds, and more preferably in a range between 600 milliseconds and 6 seconds.

The duration of the or each second heating pulse, which is also referred to as second duration, preferably is equal to or less than 1 second, and in particular is equal to or less than 100 milliseconds, and in particular is equal to or less than 40 milliseconds, and in particular is equal to or less than 20 milliseconds. Preferably, the second duration is in a range between 1 millisecond and 1 second, and more preferably in a range between 10 milliseconds and 300 milliseconds.

An interval between the beginning of two consecutive first heating pulses is equal to or less than one hour, and in particular is equal to or less than 10 minutes, and in particular is equal to or less than 1 minute, and in particular is equal to or less than 30 seconds. Preferably, the interval between the beginning of two consecutive first heating pulses is in a range between 1 second and 60 minutes, and more preferably in a range between 1 minute and 10 minutes.

An interval between the beginning of two consecutive second heating pulses is equal to or less than 1 minute, and in particular is equal to or less than 10 seconds, and in particular is equal to or less than 5 seconds. Preferably, the interval between the beginning of two consecutive second heating pulses is in a range between 100 milliseconds and 1 minute, and more preferably in a range between 1 second and 30 seconds.

Since the metal oxide material of the layer requires heating for enabling significant measurement results, it is preferred that one or more measurements are taken in response to a, and preferably each first heating pulse. Such measurement may be taken during the heating or short-after, for example. While in one embodiment, a single value indicative of the resistance of the layer - which value is also referred to as resistance value in the following, and which value shall include the resistance value itself as well as any other value indicative of the resistance of the layer - is measured in response to the first heating pulse, in a different embodiment multiple resistance values may be taken in response to the, or each first heating pulse. It is preferred, that a first concentration value is derived from the single resistance value and is output, e.g. to a user, while in the latter case an average of the multiple resistance values may be transformed into the then present first concentration value. It is preferred, that the first concentration value is determined dependent solely on the one or more resistance values measured in response to the associate first heating pulse.

In response to a, and preferably each second heating pulse, one or more resistance measurements are taken resulting in a single or multiple resistance value/s. In case of multiple resistance values those may be averaged for supporting the determination of a second concentration value. However, given that the second heating pulses are of shorter duration than the first heating pulses a measurement in response thereto may not be as exact as in response to a first heating pulse. Therefore, it is preferred that a second concentration value is determined in response to a or each of the second heating pulses dependent on at least the one or more resistance values measured during the associate second heating pulse and in addition dependent on the one or more resistance values measured in response to the previous first heating pulse. Specifically, the second concentration value may additionally be determined dependent on the one or more resistance values measured in response to every second heating pulse generated between the previous first heating pulse and the associate second heating pulse. Preferably, the second concentration value is determined dependent on the resistance value measured in response to the previous first heating pulse and dependent on a deviation of the resistance value measured in response to the associate second heating pulse from the resistance value measured in response to the previous first heating pulse. Hence, the second heating pulses may be generated for providing measurement points between the first heating pulses used for e.g. inter- or extrapolating a course of concentration assembled from the first and the second concentration values.

At some points in time, the layer may preferably be exposed to an additional heating in form of a pre-heat step. The pre-heat step may automatically be generated or may be user-initiated. In such pre-heat step the heater preferably is activated by a third electrical pulse of a third magnitude for generating a third heating pulse. The third magnitude exceeds the first magnitude. A rate at which the third heating pulses are generated is preferably lower than a rate at which first heating pulses are generated. Hence, between two consecutive third heating pulses multiple first heating pulses are generated, e.g. preferably more than fifty first heating pulses. It was found that such pre-heat step may improve a cross-sensitivity of the measurement particularly for sulfur compounds such as hydrogen sulfide, carbon monoxide, or methane, in the presence of for example ethanol or humidity ($H_2O$). This reduced cross-sensitivity is particularly advantageous for the measurement of breath components.

It is preferred that a temperature of the layer achieved during or in response to a first heating pulse is between 20°C and 200°C, and more preferably in a range between 50°C and 150°C. The same is true for a second heating pulse. For a third heating pulse, a temperature of the layer achieved during or in response to a third heating pulse is desired to be between 200°C and 400°C, and preferably in a range between 250°C and 350°C.

Preferably, the method is applied to applications that run permanently, and therefore sense gaseous compounds for at least multiple hours. Specific applications may include the detection of toxic gases indoor - such as monitoring indoor air quality - or outdoor, including but not limited to the detection of volatile organic compounds (VOCs), carbon monoxide or methane, or the detection of other substances. Preferably, the metal oxide layer comprises preferably tin oxide, particularly tin oxide in form of nanoparticles. The tin oxide can further be doped with palladium, preferably with the doping being in the range between one and four weight percent.

A device containing a gas sensor according to any of the above embodiments can be a portable electronic device such as a smart phone, a handheld or wearable computer, a laptop, an electronic reader, a tablet computer, a game controller, a pointing device, a photo or a video camera, or a computer peripheral. Its housing is typically a shell of metal, glass, or plastic material and can be assembled as a unibody or from several parts. Enclosed in the housing are typically processors, drivers for parts such as screens, antennae, cameras, microphones and speakers as well as batteries to provide power to the device and its parts. A screen is typically arranged as a part of the housing or mounted behind a transparent window of the housing. In other embodiments, the device containing the gas sensor may be a data logger or a sensor dongle.

The gas sensor may be integrated with CMOS circuitry for control and read-out onto a common substrate.

Other advantageous embodiments are listed in the dependent claims as well as in the description below.

All described embodiments similarly pertain to the method and the device.

BRIEF DESCRIPTION OF THE FIGURES

The above and other embodiments of the present invention together with further advantages are described in further details in the following description and figures.

FIG. 1 shows a metal oxide gas sensor according to an embodiment of the present invention, in a schematic perspective view of a cut in diagram 1a), and in a schematic cross-section of the membrane in diagram 1b);

FIG. 2 illustrates an operational mode of a heater of a gas sensor according to an embodiment of the present invention; and

FIG. 3 illustrates an operational mode of a heater of a gas sensor according to another embodiment of the present invention.

DETAILED DESCRIPTION

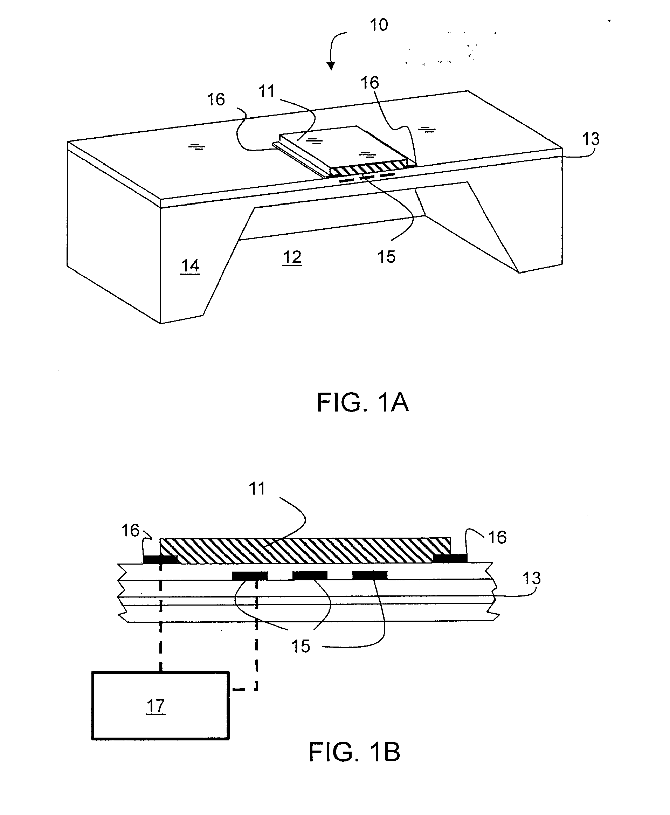

A gas sensor 10 is shown in Figs 1A and 1B comprising a sensing layer 11 of metal oxide. The gas sensor 10 preferably is integrated with a CMOS circuitry (not shown) on a single chip. A stack of layers 13 is arranged on a semiconductor substrate 14 required for the CMOS circuitry. A portion of the semiconductor substrate 14 is etched away to form a cavity 12 at the location of the layer 11. Remaining layers 13 and possibly a remaining portion of the substrate 14 form a thin membrane to support the layer 11.

Embedded within the layers 13 are conducting elements collectively referred to as heater 15 for providing a local source of heat to heat the metal oxide layer 11 e.g. during operation of the gas sensor 10. The temperature can rise rapidly around the metal oxide layer 11 on the membrane, while a thicker part of the gas sensor chip, i.e. the portion where the substrate 14 is not removed, reacts with a slower rise of temperature due to its thermal inertia. By controlling the heater 15 accordingly, the metal oxide layer 11 can be activated for a measurement and be regenerated afterwards.

The metal oxide layer 11 is contacted by two conductive electrodes 16 and hence acts as a resistor. In the presence of a compound its resistance changes thereby providing a measure of a concentration of the compound in the immediate vicinity of the metal oxide layer 11.

Both the conductive electrodes 16 and the heater 15 are preferably connected to a control unit 17, which can be implemented as a part of the CMOS circuitry arranged on the same substrate 14. The control unit 17 may control an amount of electrical power applied to the heater 15 and may perform and register measurements of the resistance of the metal oxide layer 11.

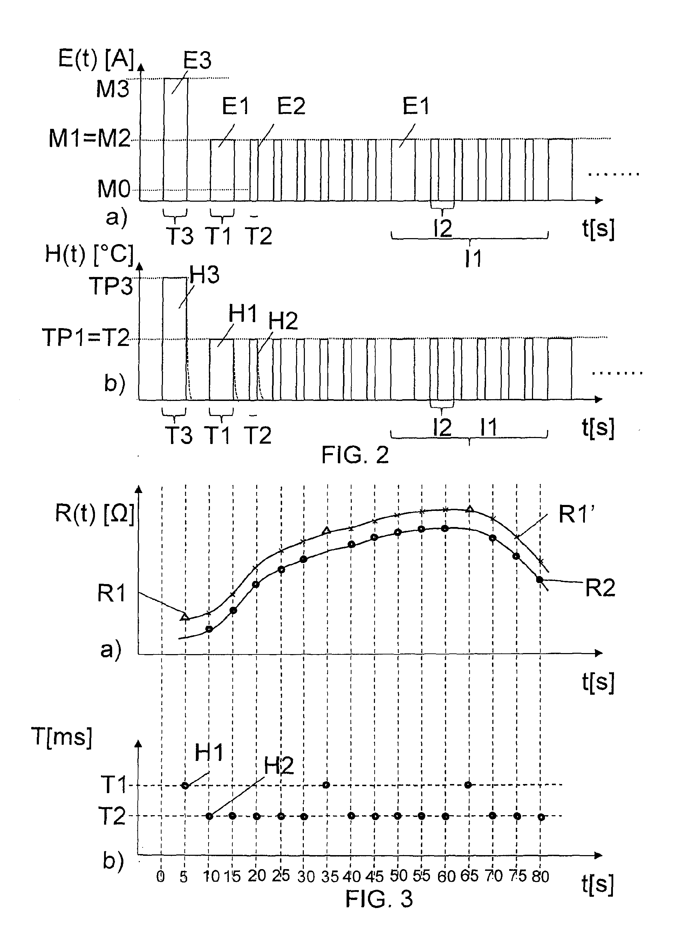

FIG. 2 illustrates an operational mode of a heater of a gas sensor according to an embodiment of the present invention, e.g. of the heater 15 of the gas sensor as shown in FIG. 1.

In diagram 2a), an electrical signal E(t) is shown over time t for driving the heater according to a first example of the present invention. The electrical signal E(t) is assumed to be a current through the resistive heater. It can be derived that three different kinds of pulses E1, E2 and E3 are contained in the electrical signal E(t), wherein first pulses E1 repeat every interval I1, and wherein second pulses E2 repeat within two consecutive first pulses E1 at intervals I2, wherein I2 < I1. First pulses E1 and second pulses E2 substantially are of the same magnitude M1 = M2, wherein "substantially" preferably encompasses a tolerance of 10 percent. A duration of the first pulse E1 is referred to as first duration T1 which exceeds a second duration T2 of a second pulse E2.

The electrical signal E(t) starts with an activation pulse which is referred to as third pulse E3 or pre-heat pulse. The third pulse E3 has a magnitude M3 that exceeds the magnitudes M1, M2 of both the first and the second pulses E1, E2. A duration T3 of the third pulse E3, is assumed to be equal to the first duration T1, however, it may also exceed the first duration T1 by far.

Diagram 2b), shows a corresponding heating pattern H(t) over time t that results from driving the heater with the electrical signal E(t) of diagram 2a). The heating pattern H(t) is assumed to follow closely the electrical signal E(t) or may show slopes as indicated by dotted lines. Hence, the heating pattern H(t) essentially corresponds in shape to the driving electrical signal E(t) and hence shows heating pulses H1, H2, H3 that correspond to the pulses E1, E2 and E3 in the electrical signal E(t). Accordingly, first heating pulses H1 are of duration T1 each and of temperature TP1 each, second heating pulses H2 are of duration T2 each and of temperature TP2 each, and third heating pulses H3 are of duration T3 each and of temperature TP3 each which temperature TP3 exceeds both temperatures TP1 and TP2, and wherein the first duration T1 exceeds the second duration T2. The layer 11 of metal oxide is exposed to the heating pulses H1, H2, H3 of such property and is assumed to take the corresponding temperatures TP1, TP2, TP3.

The rationale in applying heating pulses H1 and H2 of different duration T1 and T2 is to conduct regularly measurements in response to the first heating pulses H1 that sufficiently heat the layer 11 resulting in a precise measurement value. Given that such first heating pulses H1 may be power consuming, it is envisaged to apply multiple interim second heating pulses H2 of a shorter duration T2 consuming less energy. A measurement in response to such a second heating pulse H2 may provide a resistance value of less accuracy than in response to a first heating pulse H1. However, such resistance value R2 may be sufficient for inter- or extrapolating between two resistance values R1 in response to the first longer heating pulses H1. For example, first heating pulses H1 have a first duration T1 of 150 ms each for accurate measurements while second heating pulses H2 have a second duration T2 of 20 ms each to determine relative changes in between two consecutive first heating pulses H1.

The interval I1 between two consecutive first heating pulses H1, and the number of second heating pulses H2 within two consecutive first heating pulses H1 - which is reflected by the interval I2 between two consecutive second heating pulses H2 - can be defined according to a required update rate of measurement values and / or according to power consumption.

The third heating pulse H3 instead serves as a pre-heat pulse that lowers cross-sensitivity for the measurements following. Typically, no measurement is taken in response to the third heating pulse H3.

Preferably, the first duration T1 is in a range between 100 milliseconds and 30 seconds, and more preferably in a range between 600 milliseconds and 6 seconds. Preferably, the second duration T2 is in a range between 1 millisecond and 1 second, and more preferably in a range between 10 milliseconds and 300 milliseconds. Preferably, the interval I1 between the beginning of two consecutive first heating pulses is in a range between 1 second and 60 minutes, and more preferably in a range between 1 minute and 10 minutes. Preferably, the interval I2 between the beginning of two consecutive second heating pulses is in a range between 100 milliseconds and 1 minute, and more preferably in a range between 1 second and 30 seconds.

FIG. 3 illustrates an operational mode of a heater of a gas sensor according to another embodiment of the present invention, e.g. of the heater 15 of the gas sensor as shown in FIG. 1. In diagram 3b), a sample heating pulse pattern is provided wherein a circle represents either a first heating pulse H1 of first duration T1 or a second heating pulse H2 of second duration T2. As is shown, the measurement is initialized at 5 seconds where the temperature of the metal oxide layer is raised from room temperature to the first temperature TP1 of about 150°C as measurement temperature for a first duration T1 of 150 ms. At t = 10s, 15s, 20s, 25s, and 30 s, the temperature of the metal oxide layer is raised each time from a temperature inside the device to the second temperature TP2 of 150 degrees Celsius as measurement temperature for 20 ms each. At t = 35s, the temperature is raised again for the first duration T1 to the first temperature TP1, and so on. Hence, the first heating pulses H1 of first duration T1 are applied every 30 seconds, while the second heating pulses H2 are applied every 5 seconds within two consecutive first heating pulses H1. In between the various heating pulses H1 and H2, the heater is assumed not to be activated.

Diagram 3a) illustrates corresponding measurement results over time t. It is assumed that a resistance of a metal oxide layer is measured in Ohms and it is assumed, that in the present example a single measurement is taken in response to each of the first and the second heating pulses H1, H2. A triangle in diagram 3a) represents a resistance value R1 measured in response to a first heating pulse H1 of first duration T1, while a circle refers to a resistance value R2 measured in response to a second heating pulse H2 of second duration T2. It can be derived from diagram 3a), that the resistance values R2 resulting from the second heating pulses H2 tend to show a lower value than the resistance values R1 resulting from the first heating pulses H1 given that the second heating pulses H2 are dimensioned to save energy at an expense of accuracy in the measurement result. Crosses in diagram 3a), crosses would indicate resistance values R1' that would have been measured if first heating pulses H1 would have been applied instead second heating pulses H2 which, however, is not preferred in view of energy consumption.

In response to each first heating pulse H1, a first concentration value C1 is determined dependent on the corresponding first resistance value R1 as measured in response to the first heating pulse H1, and preferably solely dependent on the corresponding first resistance value R1 as measured but not any other measured resistance value. For example, at $t = t_{H1}$, which is the time a first heating pulse H1 is applied, the first concentration value C1 is determined by

$$C1(t_{H1}) = \alpha * R1(t_{H1})^n$$

with $\alpha$ being a constant or variable;
n = 1, 2, 3 ...; and

$R1(t_{H1})$ being the resistance value R1 measured in response to the first heating pulse H1 applied at time $t_{H1}$.

There may be other terms involved in determining the first concentration value, such as terms owed to linearization, etc.

In addition, in response to each second heating pulse H2, a second concentration value C2 is determined. Here, it is preferred to additionally use the resistance value R1 taken in response to the most previous first heating pulse H1 in addition to the resistance value R2 that is taken in response to the associated, present second heating pulse H2.

In the following $t_{H1}$ is the time a first heating pulse H1 is applied, $t_{H1+H2}$ shall be the time the first second heating pulse H2 is applied following the first heating pulse H1, $t_{H1+2H2}$ shall be the time the next second heating pulse H2 is applied, and so on, whereas $t_{H1-H2}$ shall be the time the last second heating pulse H2 is applied prior to having applied the first heating pulse H1 at $t_{H1}$. And, $t_{-H1}$ shall be the time the next earlier first heating pulse H1 is applied prior to having applied the first heating pulse H1 at $t_{H1}$.

Given that it is known that in view of the short duration of the second heating pulse H2 the corresponding measured resistance value R2 will usually be less than the resistance value if the longer first heating pulse H1 would have been applied, it is preferred that a second concentration value C2 in response to a second heating pulse H2 at time $t_{H1+x*H2}$ is determined by

$$C2(t_{H1+x*H2}) = \beta * (R2(t_{H1+x*H2})^n + \Delta(t_{H1}))$$

wherein:
$\beta$ is a constant or variable;
n = 1, 2, 3 ...;
$R2(t_{H1+x*H2})$ is the resistance value R2 measured in response to this second heating pulse H2 at time $t_{H1+x*H2}$; and wherein

$$\Delta(t_{H1}) = R1(t_{H1}) - R2(t_{H1})$$

with $\Delta$ being a difference between the first resistance value R1 measured at time $t_{H1}$ in response to the previous first heating pulse H1, and a resistance value R2 that would have been measured in response to a second heating pulse H2 if hypothetically being applied at time $t_{H1}$. However, this resistance value $R2(t_{H1})$ is not known given that at time $t_{H1}$ only the long first heating pulse H1 is applied but not the short second heating pulse H2. The underlying idea is to add to the second resistance value R2 measured in response to a second heating pulse H2 the difference $\Delta$ between the previous first resistance value R1 as measured and a second resistance value R2 as if taken at the same point in time $t_{H1}$.

In one embodiment, this second resistance value R2($t_{H1}$) is determined by interpolation between the measured second resistance values R2 at times $t_{H1-H2}$ and $t_{H1+H2}$. Accordingly

$$R2(t_{H1}) = (R2(t_{H1-H2}) + R2(t_{H1+H2})) / 2$$

In an alternative, this second resistance value R2($t_{H1}$) is determined by extrapolation from the second resistance value R2 at time $t_{H1-H2}$. For example:

$$R2(t_{H1}) = R1(t_{H1}) - \Delta(t_{-H1})$$

wherein

$$\Delta(t_{H1}) = R1(t_{-H1}) - R2(t_{-H1})$$

In each of intrapolation and extrapolation, the expression log(Rx) can be applied instead of R(x), x $\in$ [1,2].

As a result, the concentration C(t) measured over time t contains in series : A first concentration value C1, multiple second concentration values C2, a first concentration value C1, and so on. In the present example of FIG. 3 the second concentration values C2 are determined at every 5 seconds while the first concentration values are determined every 30 seconds. This leads to an overall refresh rate of 5 seconds.

Summarizing, the hybrid mode as introduced uses pulses long enough to measure accurate gas concentrations at a lower rate in combination with short pulses in between to measure changes of the gas concentration at a higher frequency. The measurement results are combined to achieve an overall high accuracy with a fast refresh rate and low power consumption.

It is noted, that the electrical signal E(t) and the resulting heating pulses H(t) preferably are initiated by the control unit 17.

While there are shown and described presently preferred embodiments of the invention, it is to be understood that the invention is not limited thereto but may be otherwise variously embodied and practised within the scope of the following claims.

**Claims**

1. A method for determining a concentration of a compound in a gas by using a gas sensor (10) with a layer (11) comprising metal oxide, the method comprising
   heating the layer (11) by first heating pulses (H1) of a first duration (T1) each, and by at least one second heating pulse (H2) of a second duration (T2) between two consecutive first heating pulses (H1), which second duration (T2) is less than the first duration (T1) and is equal to or less than 5 seconds, and
   deriving the concentration of the gas compound from values indicative of a resistance of the layer (11) as measured in response to one or more of the first and second heating pulses (H1, H2).

2. The method of claim 1,
   wherein at least one value (R1, R2) indicative of a resistance of the layer (11) is measured in response to each first and each second heating pulse (H1, H2), and
   in particular wherein exactly one value (R1, R2) indicative of a resistance of the layer (11) is measured in response to each first and each second heating pulse (H1, H2).

3. The method of claim 1 or claim 2,

wherein a first concentration value (C1) is determined dependent on the one or more values (R1) indicative of a resistance of the layer (11) measured in response to an associate first heating pulse (H1).

4. The method of any of the preceding claims,
wherein a second concentration value (C2) is determined dependent on at least the one or more values (R2) indicative of a resistance of the layer (11) measured in response to an associate second heating pulse (H2) and the one or more resistance values (R1) measured in response to the previous first heating pulse (H1),
and in particular wherein the second concentration value (C2) is additionally determined dependent on the one or more values (R2) indicative of a resistance of the layer (11) measured in response to second heating pulses (H2) generated between the previous first heating pulse (H1) and the associate second heating pulse (H2).

5. The method of claim 4,
wherein the second concentration value (C2) is determined dependent on a deviation (Δ) of the value (R1) indicative of a resistance of the layer (11) measured in response to the previous first heating pulse (H1) from a value (R2) indicative of a resistance of the layer (11) as if measured in response to a second heating pulse (H2) applied at the same point in time,
wherein the value (R2) indicative of the resistance of the layer (11) as if measured in response to the second heating pulse (H2) applied at the same point in time is determined by interpolation or extrapolation.

6. The method of any of preceding claims,
wherein the second duration (T2) is equal to or less than 1 second, and in particular is equal to or less than 100 milliseconds, and in particular is equal to or less than 40 milliseconds, and in particular is equal to or less than 20 milliseconds.

7. The method of any of the preceding claims,
wherein the first duration (T1) is equal to or less than 10 minutes, and in particular is equal to or less than 1 minute, and in particular is equal to or less than 10 seconds, and in particular is equal to or less than 1 second, and in particular is equal to or less than 500 milliseconds, and in particular is equal to or less than 150 milliseconds.

8. The method of any one of the preceding claims,
wherein an interval (I1) between the beginning of two consecutive first heating pulses (H1) is equal to or less than one hour, and in particular is equal to or less than 10 minutes, and in particular is equal to or less than 1 minute, and in particular is equal to or less than 30 seconds.

9. The method of any one of the preceding claims,
wherein an interval (I2) between the beginning of two consecutive second heating pulses (H2) is equal to or less than 1 minute, and in particular is equal to or less than 10 seconds, and in particular is equal to or less than 5 seconds.

10. The method of any one of the preceding claims,
wherein the layer (22) is heated by means of a heater (15),
wherein the heater (15) is activated by a first electrical pulse (E1) of a first magnitude (M1) for generating each first heating pulse (H1),
wherein the heater (15) is activated by a second electrical pulse (E2) of a second magnitude (M2) for generating each second heating pulse (H2), and
wherein the first magnitude (M1) and the second magnitude (M2) differ at a maximum of thirty percent from the lower of the first and the second magnitude (M1, M2).

11. The method of any of the preceding claims,
wherein between the first heating pulse (H1) and the subsequent second heating pulse (H2) and / or between two consecutive second heating pulses (H2) the heater (15) is one of deactivated or activated at a maximum of twenty percent, and preferably at a maximum of ten percent, of the lower of the first and second magnitude (M1, M2).

12. The method of any of the preceding claims,
wherein the gas compound is selected from a group consisting of volatile organic compounds, carbon monoxide and methane.

13. The method of any of the preceding claims,
wherein the layer (11) is heated by third heating pulses (H3) of a third duration (T3) each,

wherein the heater (15) is activated by a third electrical pulse (E3) of a third magnitude (M3) for generating each third heating pulse (H3), which third magnitude (M3) exceeds the first magnitude (M1),

wherein between two consecutive third heating pulses (H3) multiple first heating pulses (H1) are generated,

and in particular wherein more than fifty first heating pulses (H1) are generated between two consecutive third heating pulses (H3).

14. A gas sensor (10), comprising

a layer (11) comprising metal oxide,

a heater (15) arranged to heat the layer (11), and

a control unit (17) adapted to control the heater (15) to heat the layer (11) by first heating pulses (H1) of a first duration (T1) each, and by at least one second heating pulse (H2) of a second duration (T2) between two consecutive first heating pulses (H1), which second duration (T2) is less than the first duration (T1) and is less than 5 seconds, wherein the control unit (17) is adapted to derive a concentration of a gas compound supplied to the layer (11) from values (R1, R2) of the layer (11) indicative of its resistance as measured in response to one or more of the first and second heating pulses (H1, H2).

15. An electronic device comprising a gas sensor in accordance with claim 14.

FIG. 1A

FIG. 1B

FIG. 2

FIG. 3

Europäisches
Patentamt

European
Patent Office

Office européen
des brevets

# EUROPEAN SEARCH REPORT

**Application Number**

EP 15 00 0250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2009/068405 A1 (SIEMENS AG [DE]; STAHL PETER [CH]; FLEISCHER MAXIMILIAN [DE]; HOEFER U) 4 June 2009 (2009-06-04)<br>* page 3, lines 4-8, 17-28 *<br>* page 5, lines 9-11,18-37 *<br>* page 6, lines 1-7, 35-38 *<br>* page 12, lines 6-35; figures 7,8,10 * | 1-14 | INV.<br>G01N27/12 |
| A | US 2011/174799 A1 (ALI SYED ZEESHAN [GB] ET AL) 21 July 2011 (2011-07-21)<br>* paragraphs [0041], [0042] * | 1-14 | |
| A | US 2014/216129 A1 (SCHMIDLIN ROGER [CH] ET AL) 7 August 2014 (2014-08-07)<br>* paragraph [0068] * | 1-14 | |
| A | JAEGLE M ET AL: "Micromachined thin film SnO2 gas sensors in temperature-pulsed operation mode",<br>SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, CH,<br>vol. 57, no. 1-3,<br>7 September 1999 (1999-09-07), pages 130-134, XP004251733,<br>ISSN: 0925-4005, DOI:<br>10.1016/S0925-4005(99)00074-X<br>* the whole document * | 1-14 | |

| | | | TECHNICAL FIELDS SEARCHED (IPC) |
|---|---|---|---|
| | | | G01N |

The present search report has been drawn up for all claims

| Place of search | Date of completion of the search | Examiner |
|---|---|---|
| Munich | 30 June 2015 | Stussi, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 00 0250

This annex lists the patent family members relating to the patent documents cited in the above-mentioned European search report.
The members are as contained in the European Patent Office EDP file on
The European Patent Office is in no way liable for these particulars which are merely given for the purpose of information.

30-06-2015

| Patent document cited in search report | | Publication date | Patent family member(s) | | Publication date |
|---|---|---|---|---|---|
| WO 2009068405 | A1 | 04-06-2009 | DE 102007057519 A1<br>WO 2009068405 A1 | | 01-10-2009<br>04-06-2009 |
| US 2011174799 | A1 | 21-07-2011 | NONE | | |
| US 2014216129 | A1 | 07-08-2014 | CN 103970529 A<br>EP 2762881 A1<br>US 2014216129 A1 | | 06-08-2014<br>06-08-2014<br>07-08-2014 |

EPO FORM P0459

For more details about this annex : see Official Journal of the European Patent Office, No. 12/82